# EUROPÄISCHE PATENTANMELDUNG

(11) **EP 1 464 945 A2**
(43) Veröffentlichungstag der Anmeldung: **06.10.2004**
(21) Anmeldenummer: 04008210.9
(22) Anmeldetag: 05.04.2004
(51) Int. Cl.: G01N 1/22, B01L 3/00

(54) **Becher und Verfahren zur Bereitstellung von Geruchsproben**

(30) Priorität: 04.04.2003 DE 20305431 U
(71) Anmelder: Olfatec GmbH, 24211 Honigsee (DE)
(72) Erfinder: Björn Maxeiner, 24107 Kiel (DE)
(74) Vertreter: Seemann, Ralph, Dr. Dipl.-Phys.

(57) **Zusammenfassung**

Die Erfindung betrifft einen Becher (1) mit einem Deckel (4) zum Verschließen eines eine Geruchsprobe aufnehmenden Raumes (6), der durch den Becher (1) und den Deckel (2) eingeschlossen ist. Ferner betrifft die Erfindung ein Verfahren zur Bereitstellung von Geruchsproben, wobei Geruchsemissionen von festen und/oder flüssigen Materialien oder Bauteilen in einem durch Deckel (4) verschließbaren Becher eingefangen werden.

Die erfindungsgemäße Vorrichtung zeichnet sich dadurch aus, dass ein Rohr (3) zur Abgabe der Geruchsprobe an die Umgebung (7) vorgesehen ist, wobei der Deckel (4) in den Becher (1) drückbar ist.

Das erfindungsgemäße Verfahren zeichnet sich dadurch aus, dass anschließend durch Hineindrücken des Deckels (4) in den Becher (1) die Geruchsprobe aus einem Rohr (3) in die Umgebungsluft (7) entweicht.

## Beschreibung

Die Erfindung betrifft einen Becher mit einem Deckel zum Verschließen eines eine Geruchsprobe aufnehmenden Raumes, der durch den Becher und den Deckel eingeschlossen ist. Die Erfindung betrifft ferner ein Verfahren zur Bereitstellung von Geruchsproben, wobei Geruchsemissionen von festen und/oder flüssigen Materialien oder Bauteilen in einem durch Deckel verschließbaren Becher eingefangen werden.

Die Erfindung findet Anwendung bei der Beurteilung und Bewertung von Gerüchen. Gemäß dem Stand der Technik werden üblicherweise Behältnisse zur Aufbereitung und Darbietung von Geruchsproben durch Deckel verschlossen, die nicht geeignet sind, um die Probenluft gezielt heraus zu transportieren. So werden nach einer weit verbreiteten Richtlinie VDA270 (Verband der Automobilindustrie e.V.) verschraubbare Deckel verwendet. Andere Automobilhersteller verwenden Exsikkatoren, bei denen ein Deckel zur Probendarbietung lediglich zur Seite geschoben wird. Exsikkatoren sind dadurch gekennzeichnet, dass die Auflagefläche des Glasbehältnisses und des Glasdeckels einen Planschliff aufweisen. Aufgrund des Planschliffes ist das Glasbehältnis im Gleichdrucksystem gasdicht.

Es ist Aufgabe der vorliegenden Erfindung eine Vorrichtung und ein Verfahren anzugeben, mit dem es möglich ist, geruchliche Emissionen von festen und/oder flüssigen Materialien oder Bauteilen zunächst einzufangen und dann gezielt Probanden darzubieten.

Gelöst wird diese Aufgabe durch einen Becher mit einem Deckel zum Verschließen eines eine Geruchsprobe aufnehmenden Raumes, der durch den Becher und den Deckel eingeschlossen ist und mit einem Rohr zur Abgabe der Geruchsprobe an die Umgebung, wobei der Deckel in den Becher drückbar ist. Hierdurch wird erreicht, dass die in dem Becher generierte Geruchsprobe sich aus dem Becher heraustransportieren lässt, ohne dass sich die Probenluft mit der Umgebung verdünnt. Hierdurch bleibt die zu beurteilende Geruchsstoffkonzentration für alle nacheinander folgenden Probanden konstant und wird nicht verändert.

Vorzugsweise führt das Rohr durch den Deckel. Wenn eine Führung für das Rohr vorgesehen ist, können Undichtigkeiten zwischen Deckel und Becher vermieden werden, da so ein seitliches Verkippen des Rohres vermieden wird. Hierzu ist vorzugsweise eine Abdeckung des Bechers als Führung ausgebildet. Wenn der Deckel mit einer Dichtung ausgebildet ist, können Toleranzen des Bechers ausgeglichen werden.

Die Aufgabe wird ferner durch ein Verfahren zur Bereitstellung von Geruchsproben gelöst, wobei Geruchsemissionen von festen und/oder flüssigen Materialien oder Bauteilen in einem durch Deckel verschließbaren Becher eingefangen werden und anschließend durch Hineindrücken des Deckels in den Becher die Geruchsprobe aus einem Rohr in die Umgebungsluft entweicht. Hierdurch verändert sich die Geruchsstoffzusammensetzung, die sich in dem mit Deckel verschlossenen Becher befindet, von Proband zu Proband nicht. Vorzugsweise wird der Deckel durch Handhabung des Rohrs in den Becher gedrückt.

Bei bisher verwendeten Behältnissen kommt es zu einer unkontrollierten Verdünnung der Probenluft mit der Umgebungsluft. Durch diese Verdünnung verändert sich die Geruchskonzentration der zu messenden Probenluft. Der jeweils nachfolgende Proband, der die vermeintlich selbe Probe aus demselben herkömmlichen Behältnis bewertet, findet tatsächlich eine andere Geruchsstoffzusammensetzung vor, als der vorangegangene Proband. Damit werden die Messungen ungenau und die Ergebnisse verfälscht. Dieses Problem wird von den erfindungsgemäßen Merkmalen gelöst.

Mit der von den Ansprüchen umfassten Lösung wird erreicht, dass die in dem Becher, insbesondere Becherglas, generierte Geruchsprobe sich aus dem Becher bzw. Becherglas heraustransportieren lässt, ohne dass sich die Probenluft mit der Umgebung verdünnt. Damit bleibt die zu beurteilende Geruchsstoffkonzentration für alle nacheinander folgenden Probanden konstant und wird nicht verändert.

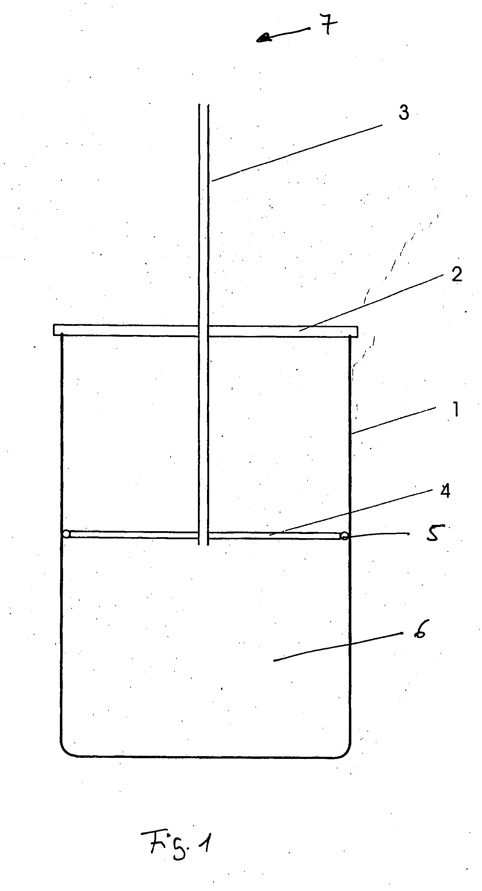

Im Folgenden wird anhand der Fig. 1 ein Ausführungsbeispiel der Erfindung erläutert.

Maßgeblich für die Funktionalität ist die Sicherstellung, dass sämtliche Materialien, die mit der Geruchsprobenluft in Berührung kommen, mit dieser Probe nicht reagieren. Die DIN EN 13725 definiert für solch ein Einsatzgebiet freigegebene Materialien. Die hier verwendeten Materialien Glas, PTFE und Edelstahl sind freigegeben. Das hier verwendete 5l Becherglas 1 ist ein handelsüblicher Glasbehälter. Der aus Edelstahl gefertigte Deckel 4 wird durch eine PTFE-Dichtung 5 hin zum Becherglas 1 abgedichtet, um Toleranzen des Becherglases 1 auszugleichen. Das Edelstahlrohr 3 führt durch den Deckel 4 und ist durch eine Schraubverbindung fest mit dem Deckel 4 verbunden. Als Führung für das Edelstahlrohr 3 dient eine Abdeckung 2 auf dem Becherglas 1. Die Führung verhindert ein seitliches Verkippen des Edelstahlrohrs bzw. Deckelsystems 3 und 4, so dass Undichtigkeiten zwischen Deckel 4 und Becherglas 1 vermieden werden. Die Geruchsprobe ist in dem Raum'6, der durch den Deckel 4 und das Becherglas 1 eingeschlossen bzw. definiert ist, enthalten. Durch Hineindrücken des Deckels 4 in das Becherglas 1 entweicht ein Teil der Geruchsprobe, die sich in dem Raum 6 befindet, durch das Rohr 3 in die Umgebungsluft 7 und kann so von Probanden bewertet werden.

### Bezugszeichenliste

- 1: Becher
- 2: Abdeckung/Führung
- 3: Edelstahlrohr für Deckel
- 5: Dichtung
- 6: Raum
- 7: Umgebungsluft

## Patentansprüche

1. Becher (1) mit einem Deckel (4) zum Verschließen eines eine Geruchsprobe aufnehmenden Raumes (6), der durch den Becher (1) und den Deckel (2) eingeschlossen ist, und mit einem Rohr (3) zur Abgabe der Geruchsprobe an die Umgebung (7), wobei der Deckel (4) in den Becher (1) drückbar ist.

2. Behälter nach Anspruch 1, **dadurch gekennzeichnet, dass** das Rohr (3) durch den Deckel (4) führt.

3. Behälter nach Anspruch 1 oder 2, **dadurch gekennzeichnet, dass** eine Führung (2) für das Rohr (3) vorgesehen ist.

4. Behälter nach Anspruch 3, **dadurch gekennzeichnet, dass** eine Abdeckung (2) des Bechers (1) als Führung (2) ausgebildet ist.

5. Behälter nach einem oder mehreren der Ansprüche 1 bis 4, **dadurch gekennzeichnet, dass** der Deckel (4) mit einer Dichtung (5) ausgebildet ist.

6. Behälter nach Anspruch 5, **dadurch gekennzeichnet, dass** die Dichtung (5) am Umfang des Deckels (4) angeordnet ist.

7. Verfahren zur Bereitstellung von Geruchsproben, wobei Geruchsemissionen von festen und/oder flüssigen Materialien oder Bauteilen in einem durch Deckel (4) verschließbaren Becher (1) eingefangen werden und anschließend durch Hineindrücken des Deckels (4) in dem Becher (1) die Geruchsprobe aus einem Rohr (3) in die Umgebungsluft (7) entweicht.

8. Verfahren nach Anspruch 7, **dadurch gekennzeichnet, dass** der Deckel (4) durch Handhabung des Rohres (3) in den Becher (1) gedrückt wird.
